(19) **Europäisches Patentamt**

**European Patent Office**

**Office européen des brevets**

(11) **EP 1 550 711 A1**

(12) **EUROPEAN PATENT APPLICATION**
published in accordance with Art. 158(3) EPC

(43) Date of publication:
**06.07.2005 Bulletin 2005/27**

(51) Int Cl.⁷: **C11D 3/386**, C11D 3/395,
C12N 9/08

(21) Application number: **03755681.8**

(22) Date of filing: **24.09.2003**

(86) International application number:
**PCT/JP2003/012143**

(87) International publication number:
**WO 2004/033610 (22.04.2004 Gazette 2004/17)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **08.10.2002 JP 2002294528**

(71) Applicant: **Mitsui Chemicals, Inc.**
**Tokyo 105-7117 (JP)**

(72) Inventors:
• **MORITA, Miyuki**
  **Hokkaido University of Ed. Sapporo**
  **Sapporo-shi, Hokkaido 002-8502 (JP)**
• **ITO, Kiyoshi**
  **Sodegaura-shi, Chiba 299-0265 (JP)**

(74) Representative: **Paget, Hugh Charles Edward et al**
**Mewburn Ellis LLP**
**York House**
**23 Kingsway**
**London WC2B 6HP (GB)**

(54) **METHOD OF USING RICE-DERIVED PEROXIDASE**

(57) The present invention provides a method of using a rice peroxidase, particularly rice hull peroxidase, having excellent reactivity at high temperatures and reactivity.

Dyed substances (dyes, etc.) are decomposed (discolored/bleached) in a washing liquor by means of a rice peroxidase. More particularly, in the case of washing or rinsing a portion with another portion in the dyed fabric with the same washing liquor, the transfer of textile dyes from the dyed fabric to the other fabric is inhibited. Further, textile dyes in a solution or a dispersion solution are bleached.

The invention allows excellent prevention of dye transfer in washing or rinsing and thus provides a method better than bleaching textile dyes in a solution or a dispersion solution, and further a composition for prevention of dye transfer and for bleaching.

EP 1 550 711 A1

## Description

Technical Filed

The present invention relates to a method of using a rice-derived peroxidase (hereinafter, referred to rice peroxidase), more particularly a rice hull peroxidase, having excellent reactivity and stability at high temperatures.

More specifically, the invention is to provide a method of decomposing (discoloring and/or bleaching) a coloring substance (a dye, etc.) in a solution using a rice peroxidase, and to a composition therefor.

Thus, the invention relates to a method of preventing the transfer of a textile dye from one dyed fabric to another fabric in the case of washing and/or rinsing a number of fabrics including a dyed fabric together in a washing liquor (hereinafter, referred to as a method of inhibiting dye transfer). Moreover, the invention relates to a method of bleaching a textile dye in a solution or a dispersion solution. Further, the invention relates to a detergent composition or a bleaching composition comprising a rice peroxidase.

Background Art

Peroxidases (E.C. 1.11.1.7) are widely distributed in plant tissues, animal tissues, blood and the like, and it has been confirmed that several microorganisms also secrete the enzymes. These enzymes belong to a family of enzymes that act as a catalyst for the oxidation of a substrate (electron or proton donors) by means of hydrogen peroxide. Although the peroxidases are classified into several types depending on variances in their prosthetic groups and the reaction modes, industrially it is most widely known to use the heme peroxidase including a heme as a prosthetic group.

Now, the above-mentioned heme peroxidase will be explained. The heme peroxidase is a heme protein comprising a trivalent iron ion and is an enzyme catalyzing the oxidation of various compounds in the presence of hydrogen peroxide or organic peroxide. As to the mechanism thereof, it is explained that the compound catalyzes the oxidative dehydrogenation of various compounds in the presence of peroxide, as shown in the following step 1 to step 3:

Step 1:

$$\text{Peroxidase} + H_2O_2 \rightarrow \text{Complex I}$$

Step 2:

$$\text{Complex I} + AH \text{ (a reducing donor)} \rightarrow \text{Complex II} + A \text{ (an oxidized donor)}$$

Step 3:

$$\text{Complex II} + AH \rightarrow \text{Peroxidase} + A + 2H_2O$$

As representative examples of the heme peroxidase, for example, a horseradish peroxidase, a soybean peroxidase, a *Corprinus cinerus* peroxidase (see, for example, JP-A-03-1949) and the like are well known.

There have been a number of suggestions on the industrial application of peroxidases, including inter alia the use in the process of decomposing a variety of organic compounds. Examples thereof include the use of the enzymes in the process of producing pulps for paper manufacturing and the use intended to bleach and/or decompose a textile dye. As such, based on the above-described mechanism, peroxidases are usually used together with hydrogen peroxide or any compound which can generate hydrogen peroxide in situ or with an enzyme system in these known processes.

In view of the bleaching and/or decomposing the textile dye, suggestions have been made on the use of peroxidases as a dye transfer inhibiting agent and as a textile dye bleaching agent. The "inhibition of dye transfer" as used herein means a process of inhibiting transfer of a textile dye from one dyed fabric to another fabric during the washing of fabric, as described in the Japanese Patent Publication No. 2801398, for example. This problem of dye transfer is important when dark-colored fabric from which dyes percolate during washing are washed together with white or pale-colored fabric. Moreover, based on the bleaching action of peroxidases against textile dyes in a solution or a dispersion solution, said enzymes are expected to be effective in the waste water treatment in textile industries. Further, it is also known that such capability of peroxidases becomes particularly conspicuous when combined, in addition to hydrogen peroxide, with an oxidative substrate other than dyes, also referred to as an activator.

Unfortunately, the activity level of said peroxidases in the natural environment is often not maintained during relatively non-natural washing processes or in the case of wastewater treatment. In particular, it is hard to say that a variety of properties possessed by peroxidases such as reactivity (reaction rate), stability against heat, stability against pH, stability against oxidation, specificity on substrate and the like are essentially optimal for the use of the enzymes in an environment other than the natural environment. In fact, as the peroxidases which are effective for the dye transfer inhibition and the bleaching, horseradish peroxidases, soybean peroxidases, or those peroxidases such as *Corprinus cinerus* peroxidases, which are produced by microorganisms such as bacteria, yeasts and fungi, have been already suggested, but all of these have not yet reached the practical level for its properties. In particular, although textile dye transfer is likely to occur when washing is carried out at a temperature of 40°C or higher, a peroxidase exhibiting excellent reactivity and stability at such a temperature is not known.

Further, a peroxidase showing excellent stability both during the processing as a washing compositions such as granulation, etc. and during storage after the processing, is not known.

The development of these days in the genetic engineering field allows substitution of a specific amino acid residue with another amino acid residue in the amino acid sequence of a protein, such substitution being relatively easy. Further, it is possible to change the original enzyme functions to various extents, according to the position of the substituted amino acid residue, the chemical properties or physical size of the substituted amino acid residue or the like, and in some cases it is also possible to deactivate the original enzyme. Such approach can also be made on peroxidases, and for example, in JP-A-9-503664, it is attempted to improve the performance of a *Corprinus cinerus* peroxidase by modifying its amino acid sequence under the purpose of obtaining a peroxidase with enhanced efficacy in a washing environment, unfortunately without achieving sufficient performance.

Further, in order to make use of peroxidases in the inhibition of dye transfer or wastewater treatment, it is required that peroxidases *per se* be produced at low costs. That is, it is essential either to separate, purify and recover peroxidases from certain materials which contain peroxidases in large amounts, which are easily available and abundant, and which are inexpensive, or to produce said enzymes efficiently from microorganisms such as bacteria, yeast, fungi and the like.

As discussed above, since peroxidases are present in a wide range of plants (tissues), extraction from plants (tissues), with no limitation in their species, may be recommended . Horseradish is well known as a representative example. However, although horseradish contains a large amount of peroxidases, it is difficult to view that said plant is a material which is easily available and abundant, and which is inexpensive per se.

Moreover, with the recent progress in the genetic engineering technology, it is possible to produce peroxidases efficiently from microorganisms such as bacteria, yeast, fungi and the like. In this case, from the viewpoint of maintaining high production efficiency and restricting spread of genetically recombined organisms, it is necessary to control strictly the growth environment for the host microorganisms during the production of the enzymes. However, in order to control the growth environment strictly, since a device or equipment for exclusive use, which is very tightly sealed and sterilizable, and a facility for exclusive use to integrate the device or equipment are required, it is not necessarily easy to produce the enzymes at low costs.

Rice also contains peroxidases, and it has been already reported that especially rice hulls contain a relatively large amount of peroxidases (Japan Crop Society, Tokai Section Research Presentation Gazette, Vol.59, p.6). Further, although proposals have been already made on the method of purifying the enzymes (JP-A-5-22510) and on the method of using the enzymes as a plant growth promoter (JP-A-7-91169), but reports on their use for inhibition of dye transfer or bleaching have not yet been made.

Disclosure of the Invention

In view of such circumstances, a task of the present invention is to find a peroxidase that enables the expression of the activity under more non-natural conditions in which a conventional peroxidase does not act, and to provide a method of using the enzyme.

Further, an object of the invention is to provide the use of a peroxidase which has excellent activity level effective in the inhibition of dye transfer and the bleaching of dyes in a solution or a dispersion solution as well as during wastewater treatment in textile industries, and which is also cheap.

Rice hulls are generated in an annual amount of approximately two million tons in Japan, and close to 30% thereof is being either incinerated or discarded (see "Biomass Energy Environment IPC "). That is, rice hulls are a material containing a large amount of peroxidases, which is easily available and abundant, and which is inexpensive per se.

Surprisingly, the inventors found that the rice hull peroxidase is excellent in reactivity and stability at high temperatures compared with the above-mentioned horseradish peroxidase, soybean peroxidase and *Corprinus cinerus* peroxidase, and then confirmed that it is a peroxidase which enables the maintenance of the effective activity level for dye transfer inhibition and bleaching, to complete the invention. Further, the rice hull peroxidase also has an important advantage that it does not cause any significant color degradation in dyed fabric itself.

That is, by using the rice hull peroxidase which exhibits reactivity at high temperatures and stability, there can be expected the effects of the inhibiting of transfer of textile dyes and the bleaching at a wide temperature ranging from room temperature to elevated temperatures, and at the same time high stability during the processing of the enzyme as a detergent composition such as granulation, etc., and during storage after the processing.

Thus, the invention relates to the following:

[1] A method of preventing the transfer of a textile dye from one dyed fabric to another fabric in the case of washing and/or rinsing a number of fabrics including dyed fabric together in a washing liquor, wherein a rice peroxidase acts on the washing liquor in the presence of hydrogen peroxide.

[2] A method of bleaching a textile dye in a solution or a dispersion solution, wherein a rice peroxidase acts on the solution or dispersion solution in the presence of hydrogen peroxide.

[3] The method according to [1] or [2], wherein the rice peroxidase is a heat-resistant rice peroxidase.

[4] The method according to any one of [1] to [3], wherein the rice peroxidase is a rice hull peroxidase.

[5] The method according to any one of [1] to [4], wherein the hydrogen peroxide is supplied by at least one means of a hydrogen peroxide precursor, a combined system of a hydrogen peroxide precursor and of a bleaching activator which can generate hydrogen peroxide by acting on the hydrogen peroxide precursor, and an enzyme system which can generate hydrogen peroxide.

[6] The method according to [5], wherein the hydrogen peroxide precursor is perborate or percarbonate.

[7] The method according to any one of [1] to [6], which is carried out in the presence of an activator.

[8] The method according to any one of [1] to [7], which is carried out in the presence of a surfactant.

[9] The method according to any one of [1] to [8], wherein the textile dye is a synthetic dye, a natural dye or a natural-equivalent dye.

[10] A composition comprising a rice peroxidase.

[11] The composition according to [10], which is a detergent composition or a bleaching composition.

[12] The composition according to [10] or [11], which comprises at least one of hydrogen peroxide, one or more kinds of hydrogen peroxide precursors, and one or more kinds of enzyme systems which can generate hydrogen peroxide.

[13] A composition comprising a rice peroxidase, a hydrogen peroxide precursor and one or more kinds of bleaching activators which can generate hydrogen peroxide by acting on the hydrogen peroxide precursor.

[14] The composition according to any one of [10] to [13], which comprises one or more kinds of the surfactants and one or more kinds of the activators together.

According to the invention, a peroxidase is provided which has excellent reactivity and stability in the inhibition of dye transfer in the process of washing or rinsing, and in the bleaching of textile dyes in a solution or a dispersion solution as well as in the wastewater treatment in textile industries, and which is inexpensive.

Brief Description of Drawings

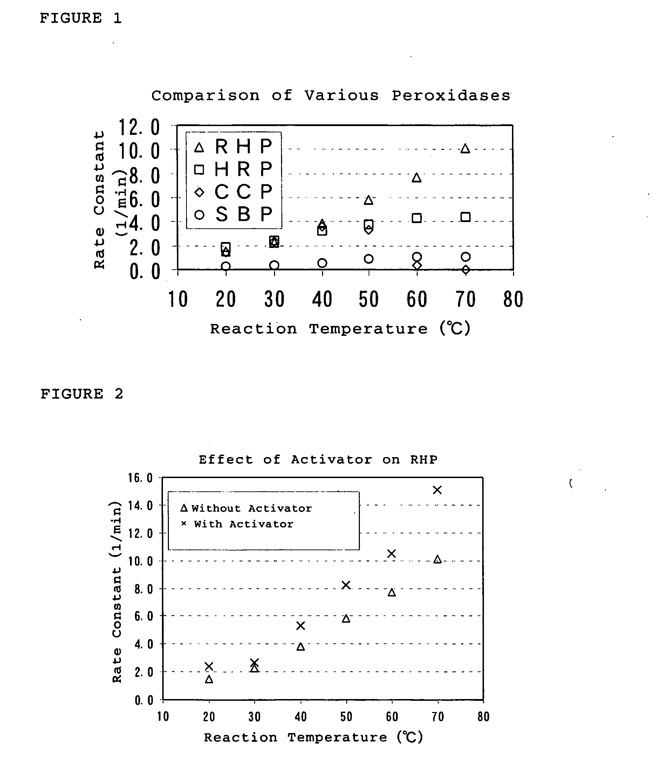

Fig. 1 shows the comparative results obtained by various peroxidases with respect to the relationship between a temperature and a reaction rate. In the figure, triangle: RHP represents a rice hull peroxidase, square: HRP represents a horseradish peroxidase, rhombus: CCP represents a *Corprinus cinerus* peroxidase, and circle: SBP represents a soybean peroxidase, respectively.

Fig. 2 shows the effect of the addition of an activator (p-iodophenol) in the discoloration reaction of Orange II by a rice hull peroxidase. In the figure, RHP represents a rice hull peroxidase. $\times$ represents addition of an activator, and triangle represents non-addition of an activator.

Best Mode for Carrying Out the Invention

The present invention will be explained in detail in the following.

The invention relates to a method of preventing the transfer of a textile dye from one dyed fabric to another fabric during washing and/or rinsing fabrics together in a washing liquor, wherein in a washing liquor which washes and/or rinses the fabrics, a rice peroxidase, and any one of hydrogen peroxide, a hydrogen peroxide precursor, a combined system of a hydrogen peroxide precursor and of a bleaching activator which can generate hydrogen peroxide by acting on the hydrogen peroxide precursor, and an enzyme system which can generate hydrogen peroxide, are present substantially simultaneously.

Further, the invention relates to a method of bleaching a textile dye in a solution or a dispersion solution , wherein in the solution or dispersion solution, a rice peroxidase and any one of hydrogen peroxide, a hydrogen peroxide precursor, a combined system of a hydrogen peroxide precursor and of a bleaching activator which can generate hydrogen peroxide by acting on the hydrogen peroxide precursor, and an enzyme system which can generate hydrogen peroxide, are present substantially simultaneously.

Moreover, the invention provides a detergent composition or a bleaching composition which contains a rice peroxidase substantially simultaneously with any one of hydrogen peroxide, a hydrogen peroxide precursor, a combined system of a hydrogen peroxide precursor and of a bleaching activator which can generate hydrogen peroxide by acting on the hydrogen peroxide precursor, and an enzyme system which can generate hydrogen peroxide.

The rice peroxidase according to the invention may contain various quantities or types of impurities that are associated with said enzyme depending on the degree of purification, but it is preferable to use an enzyme having an RZ value of 0.01 or more, more preferably 0.1 or more, and even more preferably 1.0 or more. The term RZ value as used herein is the ratio of the absorbances at 275 nm and 403 nm ($A_{275nm}/A_{403nm}$). This value indicates the ratio of the heme content to the protein content in a peroxidase solution and thus serves as an index to the degree of purification of the peroxidase.

The heat-resistant rice peroxidase according to the invention may be represented by a rice hull peroxidase which is subjected to separation, purification and recovery from rice hulls containing the enzyme, but as long as the rice peroxidase is heat-resistant, there is no particular limitation on the part of rice for the localization of the enzyme. For example, even for peroxidases derived from rice bran, stalk and leaves, as long as they are heat, they can be used as the heat-resistant rice peroxidases as mentioned in the invention. According to the invention, said enzyme is considered to have heat resistance if 50% or more of the activity is preserved even after it is subjected to a heat treatment at 50°C or higher for 30 minutes. However, the most appropriate example would be the case wherein 90% or more of the activity is preserved after the enzyme is subjected to a heat treatment at 90°C for 30 minutes, as shown in the following Examples.

The rice peroxidase, in particular the rice hull peroxidase, according to the invention can be easily obtained from rice hulls by means of the purification method described in JP-A-5-22510. Also, it can be purchased as a reagent from Funakoshi Co., Ltd.

Moreover, it has been reported, based on the recent results in the analysis of rice genome, that plural peroxidase genes are existent in rice (Chen ZH et al.; Sheng Wu Hua Xue Yu Sheng Wu Wu Li Xue Bao (Shanghai) 2001; 33(2):163-172). That is, by using the genetic engineering technology of these days, it is possible to build a recombinant DNA molecule in which a DNA sequence encoding such rice peroxidase and a DNA sequence having the function intended to express the DNA sequence encoding said peroxidase are combined, and then to transform a host cell or a host plant with said recombinant DNA molecule, to produce a rice peroxidase efficiently. When such technical background as described above is followed, a rice peroxidase which can be obtained by culturing transformed host cells or growing transformed host plants, and subjecting the resultant culture product, cultured cells or plants to a treatment to separate, purify and recover the peroxidase, is included in the scope of the rice peroxidase of the invention.

There is no particular limitation on the form of the rice peroxidase according to the invention, but upon considering the cases of the enzyme being added as a component of a detergent composition or a bleaching composition, it is preferable to be in the form of a dust-free granule or a liquid enzyme preparation. The dust-free granule can be prepared as described in the publications of US 4,106,991 and 4,661,452 and optionally coated according to any method known in the pertinent art. On the other hand, the liquid enzyme preparation can be stabilized according to a known method by the addition of polyols, for example, propylene glycols, sugars or sugar alcohols, lactic acid, boric acid or the like.

According to the invention, in the case of washing and/or rinsing fabrics together in a washing liquor, or in the case of bleaching textile dyes in a solution or a dispersion solution, 0.001 mg to 1000 mg of the rice-peroxidase may be added to one liter of such washing liquor or bleaching liquor. Furthermore, the process may be carried out at a temperature in the range of 10°C to 80°C, preferably in the range of 20°C to 70°C, and more preferably in the range of 20°C to 60°C. Moreover, the pH of the washing liquor or the bleaching liquor may be in the general pH range, and it may be the general pH range for washing and/or rinsing, that is, pH 6.5 to 10.5, preferably 6.5 to 9.5, and more preferably 7.5 to 9.5.

According to the invention, when the inhibition of dye transfer in the process of washing and/or rinsing, and the bleaching of textile dyes in a solution or a dispersion solution are carried out, it is essential that the rice peroxidase and hydrogen peroxide are present substantially simultaneously. Here, the hydrogen peroxide may be added in an amount of 0.001 to 5 mM, and preferably 0.01 to 1 mM in the beginning of the process of washing and/or rinsing or during the process.

With regard to the supply of hydrogen peroxide, hydrogen peroxide itself may be added to the washing liquor, the solution or the dispersion solution , or a hydrogen peroxide precursor that generates hydrogen peroxide in situ may be added.

Representative examples of the hydrogen peroxide precursor include perborates, percarbonates, peroxycarboxylic acid or salts thereof. More specifically, mention may be made of sodium perborate or sodium percarbonate.

Further, a bleaching activator which can generate hydrogen peroxide by acting on the hydrogen peroxide precursor may be added together with the hydrogen peroxide precursor. Such bleaching activator may include, specifically, a sodium salt of nonanoyloxybenzenesulfonic acid, a sodium salt of dodecanoyloxybenzenesulfonic acid, 4-decanoyloxybenzoic acid, tetraacetylethylenediamine and the like. The bleaching activator to be co-present is suitably added in an amount of about 1 $\mu$M to 10 mM, and more preferably about 10 $\mu$M to 1 mM in the beginning of or during the process of washing and/or rinsing.

Moreover, as a source of hydrogen peroxide, a hydrogen peroxide-generating enzyme system which generates hydrogen peroxide *in situ* may be selected. A preferred hydrogen peroxide-generating enzyme system is one which is added appropriately to a detergent composition, and which acts on the substrate (hydrogen peroxide precursor) that is easily available at low prices. An example of such substrate (hydrogen peroxide precursor) is glucose, and this can supply hydrogen peroxide by using a glucose oxidase. Other suitable oxidases include a uric acid oxidase, a galactose oxidase, an alcohol oxidase, an amine oxidase, an amino acid oxidase and a cholesterol oxidase.

According to the invention, in the cases of carrying out the inhibition of dye transfer in the process of washing and/or rinsing, and of carrying out the bleaching of textile dyes in a solution or a dispersion solution, other oxidative substrates may also be added in order to enhance the effects of the inhibition of dye transfer and the bleaching of the rice peroxidase used. Such oxidative substrates are generally referred to as activators and are exemplified by p-iodophenol, L-ascorbic acid, p-aminophenol, p-aminobenzoic acid substrates, without being limited to these. Further, the amount of the oxidative substrates to be added is suitably about 1 $\mu$M to 10 mM, and more preferably about 10 $\mu$M to 1 mM.

According to the invention, in the cases of carrying out the inhibition of dye transfer in the process of washing or rinsing, and the bleaching of textile dyes in a solution or a dispersion solution, there may be also present anionic surfactants, nonionic surfactants, cationic surfactants, amphoteric surfactants, and mixtures of these surfactants. Examples of such surfactants may include long-chained fatty acid salts (soap: an anionic surfactant) such as alkyl benzenesulfonate (LAS: an anionic surfactant), $\alpha$-olefin sulfonates (AOS: an anionic surfactant), alkyl polyoxyethylene sulfates (AES: an anionic surfactant), sodium dodecylsulfate (SDS: an anionic surfactant), sodium laurate, Brij35 (a nonioinic surfactant), alkyl polyoxyethylene ether (a nonionic surfactant), alkyl trimethylammonium salts (a cationic surfactant), dialkyldimethylammonoium salts (a cationic surfactant), dimethylalkylamine oxide (an amphoteric surfactant) and the like, without being limited to these. Further, the amount of the surfactants to be added is not particularly limited, but preferably above the critical micelle concentration (CMC) of each surfactant.

There is no particular limitation on the textile dyes on which inhibition of dye transfer and bleaching are carried out by the rice peroxidase in the invention, and any conventionally used textile dyes, including the co-presence of the above-described activators, may be used to obtain the effect. The term textile dye as used herein may include a natural dye, a natural-equivalent dye which is synthetically produced but has its structure and properties similar to the natural dye, as well as a synthetic dye ranging from general azo dyes to anthraquinone dyes and others. Furthermore, the invention is also effective on a reactive dye which binds to textiles by covalent bonding.

Finally, the invention relates to a composition, and more particularly, to a detergent composition or a bleaching composition, comprising a rice peroxidase. It is essential that the enzyme exists together with hydrogen peroxide or a component intended for supplying the hydrogen peroxide, and for that purpose, any one of a hydrogen peroxide precursor substance, a bleaching activator or a hydrogen peroxide-generating enzyme system may be selected. That is, the invention relates to a detergent composition or a bleaching composition, comprising a rice peroxidase, and at least any one of hydrogen peroxide, a hydrogen peroxide precursor, a combined system of a hydrogen peroxide precursor and of a bleaching activator that can generate hydrogen peroxide by acting on the hydrogen peroxide precursor, and an enzyme system which can generate hydrogen peroxide.

There is no limitation on the amount of the rice peroxidase in the composition, but it preferably contains 0.001 mg to 1000 mg of the rice peroxidase per liter of a washing liquor, a solution or a dispersion solution.

With regard to the form, either a powder form or a liquid form may be selected appropriately for the purpose. Specifically, a dust-free granule as described above or a liquid enzyme preparation stabilized by several stabilizers may be selected. In particular, in the case of a powder form, the composition may be a composition comprising a mixed granulated substance of all components of said composition, or a composition of mixing a granulated substance of a single individual component in said composition or a mixed granulated substance of any plural components. A mixed granulated substance of any plural components as mentioned herein is, for example, a mixed granulated substance of a rice peroxidase and of a bleaching activator that can generate hydrogen peroxide by acting on a hydrogen peroxide precursor.

There is no limitation on the amount of the rice peroxidase in the bleaching composition, as in the case of a detergent composition. Further, the form of the composition is also applied similarly.

The detergent composition or the bleaching composition according to the invention may also contain, in addition to hydrogen peroxide itself, at least one component among a hydrogen peroxide precursor, a bleaching activator which can generate hydrogen peroxide by acting on a hydrogen peroxide precursor and an enzyme system which can generate hydrogen peroxide. Further, the detergent composition or the bleaching composition according to the invention may also contain an activator to enhance the bleaching activity of the rice peroxidase, or surfactants to enhance the cleaning effect.

Moreover, when the intended composition is a detergent composition, it may even further contain other detergent components that are known in the pertinent art, for example, a builder, an anti-corrosive, a sequestering agent, an anti-soil redeposition agent, a fragrance, an enzyme stabilizer, other detergent enzymes (for example, a protease, a lipase or an amylase) or the like.

The pH value of the washing liquor containing the detergent composition of the invention or the bleaching liquor containing the bleaching composition of the invention ranges from 6.5 to 12, and preferably from 6.5 to 10.5.

Next, the invention will be explained in detail with reference to the Examples described below, which are not intended to limit the invention thereby.

EXAMPLE 1

Temperature and reaction rate of the rice hull peroxidase

The relationship between the temperature and the reaction rate of the rice hull peroxidase was investigated by means of a discoloration reaction with Orange II (C.I. Acid Orange 7: available from Kanto Chemical Co. Inc.: the same hereinafter) as a substrate.

Into a cell (light path of 1 cm) of a Hitachi U-2010 Visible Spectrophotometer, 1 mL of a 0.1 M Tris-HCl buffer solution (pH 9.0) containing 100 $\mu$M Orange II and 680 nM of the rice hull peroxidase was added and preincubated at each temperature for 10 minutes. Then, 1 mL of 1.5 mM aqueous hydrogen peroxide that was preliminarily preincubated at the same temperatures was introduced to initiate the reaction. The change in the absorbance over time at 485 nm, which is the maximum absorption wavelength of Orange II, was determined.

The rice hull peroxidase used was a rice hull peroxidase product sold by Funakoshi Co. Ltd. (Product No. K0310100: a purity RZ > 1.2). Further, the molar absorbance coefficient of said enzyme was set to $1.02 \times 10^5$ $mol^{-1}dm^3cm^{-1}$ (403 nm), as is the case of horseradish. Further, Orange II was purified by recrystallization of a reagent manufactured by Wako Pure Chemicals Industries, Ltd. and used to determine a purity by paper chromatography and UV spectrum.

From the discoloration curve for Orange II, the equation for a reaction rate was analyzed, and it was found that the discoloration reaction proceeds in a first-order reaction in any case. Therefore, from the equation for the first-order reaction rate as represented by the following equation (1):

$$\text{Equation (1)} : \ln(C_0/C_t) = k_t$$

wherein, $k_t$ ($min^{-1}$) is a constant for the first-order reaction rate, $C_0$ is an initial concentration of Orange II, and $C_t$ is a concentration of Orange II at a reaction time t, the relationship between the temperature and the rate constant $k_t$ in the discoloration reaction of Orange II was determined, and the results are presented in Table 1 below.

Table 1

| Reaction Temperature | $k_t$ |
|---|---|
| 20°C | 1.52 |
| 30°C | 2.28 |
| 40°C | 3.84 |
| 50°C | 5.81 |
| 60°C | 7.70 |
| 70°C | 10.10 |
| 80°C | 11.00 |

Further, in Comparative Example 1 below, a comparison was made as illustrated in Fig. 1 with respect to the relationship between the temperature and the rate constant $k_t$, the same results (Table 2) for a horseradish peroxidase, a *Corprinus cinerus* (formerly referred to as *Arthromyces ramosus*) peroxidase, and a soybean peroxidase, and the relationship between the temperature and the rate constant $k_t$ of the rice hull peroxidase of the present Example.

As a result, the rice hull peroxidase was found to have excellent reactivity and stability at high temperatures as compared with other peroxidases.

COMPARATIVE EXAMPLE 1

Temperature and reaction rate of various peroxidases

An investigation was made on the relationships of the temperature and the reaction rate of a horseradish peroxidase, a *Corprinus cinerus* (formerly referred to as *Arthromyces ramosus*) peroxidase and a soybean peroxidase.

The same measurements were carried out following the same procedure under the same conditions as in Example 1, except that a horseradish peroxidase, a *Corprinus cinerus* peroxidase and a soybean peroxidase were used as the peroxidase. The relationships between the temperature and the rate constant $k_t$ were determined, and the results are presented in Table 2 below.

Here, the horseradish peroxidase used was a reagent manufactured by Wako Pure Chemicals Industries, Ltd. (R.Z. = 2.65), the *Corprinus cinerus* peroxidase was a reagent manufactured by Sigma Chemical Co. (R.Z. = 2.32), and the soybean peroxidase was a reagent manufactured by Sigma Chemical Co. (R.Z. = 1.11). Further, the molar absorbance coefficient for each enzyme was set to $1.02 \times 10^5$ $mol^{-1}dm^3cm^{-1}$ (403 nm).

The results of the present Comparative Example and of Example 1 were compared as shown in Fig. 1.

Table 2

| Reaction Temperature | Horseradish | *Corprinus cinerus* | Soybean |
|---|---|---|---|
| 20°C | 1.89 | 1.42 | 0.25 |
| 30°C | 2.40 | 2.30 | 0.40 |
| 40°C | 3.18 | 3.58 | 0.53 |
| 50°C | 3.78 | 3.32 | 0.88 |
| 60°C | 4.26 | 0.34 | 1.03 |
| 70°C | 4.40 | - | 1.03 |

EXAMPLE 2

Investigation of the thermal stability at 90°C

By means of a discoloration reaction with Orange II as the substrate, the thermal stability of the rice hull peroxidase at 90°C was investigated.

A 0.1 M Tris-HCl buffer solution (pH 9.0) containing 100 μM Orange II and 680 nM of the rice hull peroxidase was preincubated at 90°C for from 0 minute to 150 minutes. Then, 1 mL of said preincubated solution was added to a cell (light path of 1 cm) of a Hitachi U-2010 Visible Spectrophotometer which was preliminarily maintained at 80°C, and 1 mL of 1.5 mM aqueous hydrogen peroxide which was preliminarily preincubated at 80°C was introduced to initiate the reaction. As performed in Example 1, the relationship between the preincubation time and the rate constant $k_t$ was determined from the discoloration curve for Orange II, and the results are presented in Table 3 below.

That is to say, it was proven that the rice hull peroxidase has high heat-resistance at 90°C.

Table 3

| Preincubation Time | $k_t$ |
|---|---|
| 0 min | 11.0 |
| 10 min | 10.8 |
| 20 min | 10.8 |
| 30 min | 9.98 |
| 60 min | 9.87 |

Table 3   (continued)

| Preincubation Time | $k_t$ |
|---|---|
| 90 min | 9.51 |
| 120 min | 9.51 |
| 150 min | 9.51 |

COMPARATIVE EXAMPLE 2

Investigation of the thermal stability of various peroxidases at 90°C

By means of the discoloration reaction using Orange II as the substrate, the thermal stability at 90°C of the horseradish peroxidase and of the soybean peroxidase was investigated. Further, with respect to the *Corprinus cinerus* peroxidase, the enzyme lost its activity rapidly at 90°C and the discoloration reaction of Orange II did not proceed, and the enzyme was tested for thermal stability at 50°C.

The measurement was carried out following the same procedure under the same conditions as in Example 2 (provided that in the case of the *Corprinus cinerus* peroxidase, both preincubation and reaction were carried out at 50°C), except that the peroxidases used were the horseradish peroxidase, the *Corprinus cinerus* peroxidase and the soybean peroxidase as in Comparative Example 1. The relationship between the preincubation time and the rate constant $k_t$ was determined, and the results are presented in Table 4 below.

Table 4

| Preincubation Time | Horseradish | Soybean | *Corprinus cinerus* |
|---|---|---|---|
| 0 min | 6.08 | 1.06 | 3.32 |
| 5 min | 3.00 | - | 1.11 |
| 10 min | 0.315 | 0.855 | - |
| 20 min | 0.293 | - | 0.937 |
| 30 min | 0.189 | 0.874 | - |
| 60 min | - | 0.828 | - |
| 150 min | - | 0.743 | 0.473 |

EXAMPLE 3

Effect of the addition of an activator (p-iodophenol)

By means of a discoloration reaction using Orange II as the substrate, an investigation was made on the relationship between the temperature and the reaction rate for the rice hull peroxidase in the presence of an activator (p-iodophenol).

Into a cell (light path: 1 cm) of a Hitachi U-2010 Visible Spectrophotometer, 1 mL of a 0.1 M Tris-HCl buffer solution (pH 9.0) containing 100 μM Orange II, 100 μM p-iodophenol and 680 nM of rice hull peroxidase was added and preincubated at each temperature for 10 minutes, and then 1 mL of 1.5 mM aqueous hydrogen peroxide that was preliminarily preincubated at the same temperatures was introduced to initiate the reaction. The relationship between the temperature and the rate constant $k_t$ was determined from the discoloration curve for Orange II as in Example 1, and the results are presented in Table 5 below. Further, a comparison was made between the results of the present Example and the results of Example 1 (Table 1) for the effect of the addition of p-iodophenol (Fig. 2).

That is to say, it was proven that the reaction rate at each temperature is enhanced to 1.3 to 1.5 folds by the addition of the activator (= p-iodophenol).

Table 5

| Reaction Temperature | $k_t$ |
|---|---|
| 20°C | 2.42 |

Table 5 (continued)

| Reaction Temperature | $k_t$ |
|---|---|
| 30°C | 2.64 |
| 40°C | 5.32 |
| 50°C | 8.23 |
| 60°C | 10.50 |
| 70°C | 15.10 |

EXAMPLE 4

Comparison of the discoloration rate constants of various colorants

The discoloration reactions of various colorants by means of the rice hull peroxidase were investigated in the presence or absence of an activator (p-iodophenol).

Into a cell (light path: 1 cm) of a Hitachi U-2010 Visible Spectrophotometer, 1 mL of a 0.1 M Tris-HCl buffer solution (pH 9.0) containing 100 $\mu$M each of various colorants listed in Table 4 and 680 nM of the rice hull peroxidase was added and preincubated at 20°C for 10 minutes, and then 1 mL of 1.5 mM aqueous hydrogen peroxide that was preliminarily preincubated at the same temperature was introduced to initiate the reaction. The relationship with the rate constant $k_t$ was determined from the discoloration curve for various colorants as in Example 1, and the results are presented in Table 6 below. Here, in the case where p-iodophenol was added, it was added to the 0.1 M Tris-HCl buffer solution (pH 9.0) to 100 $\mu$M.

From the results of the experiment, the discoloratior effect of various colorants by the rice hull peroxidase wa confirmed, and it was particularly conspicuous in the presence of an activator.

Table 6

| Reaction Temperature | Wavelength for Measurement | p-Iodophenol | |
|---|---|---|---|
| | | Not Added ($k_t$) | Added ($k_t$) |
| Orange II | 485.0 nm | 1.52 | 2.42 |
| Orange I | 481.0 nm | 12.9 | 24.3 |
| Orange G | 476.0 nm | 0.0603 | 0.574 |
| Tropeolin O | 427.8 nm | 0.0653 | 0.2 |
| Arizarin Yellow R | 372.3 nm | 0.0434 | - |
| Arizarin Red S | 516.0 nm | 0.395 | 70.6 |
| Carcone | 635.6 nm | 34.2 | 65.5 |

EXAMPLE 5

Comparison of the effect of the addition of various activators

An investigation was made on the effect of the addition of various activators in the discoloration reaction of Orange II by the rice hull peroxidase.

Into a cell (light path: 1 cm) of a Hitachi U-2010 Visible Spectrophotometer, 1 mL of a 0.1 M Tris-HCl buffer solution (pH 9.0) containing 100 $\mu$M Orange II, 100 $\mu$M each of various activators listed in Table 5 and 680 nM of the rice hull peroxidase was added and preincubated at 20°C for 10 minutes, and then 1 mL of 1.5 mM aqueous hydrogen peroxide that was preliminarily preincubated at the same temperature was introduced to initiate the reaction. The relationship with the rate constant $k_t$ was determined from the discoloration curve for Orange II as in Example 1, and the results are presented in Table 7 below.

As a result, the effect of the addition of various activators in the discoloration reaction of Orange II by the rice hull peroxidase was confirmed.

Table 7

| Activator | $k_t$ |
|---|---|
| Not Added | 1.52 |
| p-Iodophenol | 2.42 |
| L-Ascorbic acid | 1.90 |
| p-Aminophenol | 1.67 |
| p-Aminobenzoic acid | 1.65 |
| Glutathione (reduced) | 1.27 |
| D-Glucose | 1.57 |

EXAMPLE 6

Investigation of the activator (= p-iodophenol) concentration

An investigation was made on the effect of the activator (p-iodophenol) concentration in the discoloration reaction by the rice hull peroxidase using Orange II and Orange G as the substrate.

Into a cell (light path: 1 cm) of a Hitachi U-2010 Visible Spectrophotometer, 1 mL of a 0.1 M Tris-HCl buffer solution (pH 9.0) containing 100 μM Orange II (or Orange G), p-iodophenol at the concentrations listed in the following Table 8 and 680 nM of the rice hull peroxidase was added and preincubated at 20°C for 10 minutes, and then 1 mL of 1.5 mM aqueous hydrogen peroxide that was preliminarily preincubated at the same temperature was introduced to initiate the reaction. The relationship between the p-iodophenol concentration and the rate constant $k_t$ was determined from the discoloration curve for Orange II as in Example 1, and the results are presented in Table 8 below. Here, the absorbance changes for Orange II and Orange G were measured at 485 nm and 476 nm, respectively.

As a result, the effect of discoloration of Orange II and Orange G by the rice hull peroxidase at various concentrations of p-iodophenol was confirmed.

Table 8

| p-Iodophenol Concentration | $k_t$ | |
|---|---|---|
| | Orange II | Orange G |
| 1000 μM | 3.89 | 2.42 |
| 200 μM | 3.71 | 2.21 |
| 100 μM | 2.42 | 0.56 |

EXAMPLE 7

Effect of the addition of a surfactant

An investigation was made on the effect of the addition of a surfactant in the discoloration reaction by the rice hull peroxidase using Orange II as the substrate.

Into a cell (light path: 1 cm) of a Hitachi U-2010 Visible Spectrophotometer, 1 mL of a 0.1 M Tris-HCl buffer solution (pH 9.0) containing 100 μM Orange II, a surfactant at the concentrations listed in the following Table 9 and 680 nM of the rice hull peroxidase was added and preincubated at 20°C or 60°C for 10 minutes, and then 1 mL of 1.5 mM aqueous hydrogen peroxide that was preincubated at the same temperature was introduced to initiate the reaction. The relationship with the rate constant $k_t$ was determined from the discoloration curve for Orange II as in Example 1, and the results are presented in Table 9 below. Here, the amount of addition of each surfactant was adjusted such that the concentration during the reaction was 1.5 times the critical micelle concentration (CMC).

As a result, the effect of discoloration of Orange II by the rice hul peroxidase in the presence of various surfactants was confirmed.

Table 9

| Surfactant (Type) | Concentration (Final Conc.) | $k_t$ | |
|---|---|---|---|
| | | 20°C | 60°C |
| Not Added | - | 1.52 | 7.70 |
| Sodium dodecyl sulfate (SDS: an anionic surfactant) | 8.55 mM | 1.20 | 6.72 |
| Alkylbenzene sulfonate (LAS: an anionic surfactant) | 7.35 mM | 1.26 | 8.38 |
| Brij35 (a nonionic surfactant) | 90 μM | 1.29 | 7.46 |
| Sodium Laurate (a soap) | 10.1 mM | 1.43 | 5.73 |

EXAMPLE 8

Effect in a model detergent system

An investigation was made on the effect of discoloration by the rice hull peroxidase using Orange II as the substrate in a model detergent system.

Into a cell (light path: 1 cm) of a Hitachi U-2010 Visible Spectrophotometer, 1 mL of a 0.1 M Tris-HCl buffer solution (pH 9.0) containing 100 μM Orange II, a model detergent component having a composition as described below, and 680 nM of the rice hull peroxidase was added and preincubated at 20°C, 40°C or 60°C for 10 minutes, and then 1 mL of 1.5 mM aqueous hydrogen peroxide that was preincubated at the same temperatures was introduced to initiate the reaction. The relationship with the rate constant $k_t$ was determined from the discoloration curve for Orange II as in Example 1, and the results are presented in Table 10 below.

Here, the individual components in the model detergent (concentration of species) are as follows:

Sodium dodecyl sulfate (SDS) : 8.6 mM
Sodium carbonate : 2.8 μM
Sodium sulfite : 1.8 mM
Sodium metasilicate : 4.1 μM
EDTA : 59 μM
Carboxymethyl cellulose : 0.0001%

Thus, the effect of discoloration of Orange II by the rice hull peroxidase in a model detergent system was confirmed at each temperature.

Table 10

| Cleaning Temperature | $k_t$ |
|---|---|
| 20°C | 1.43 |
| 40°C | 3.77 |
| 60°C | 4.16 |

EXAMPLE 9

Effect of the prevention of dye transfer to testing fabric (1)

An investigation was made on the effect of the prevention of dye transfer to testing fabric by the rice hull peroxidase in a system using Orange II as the model textile dye.

White clothing fabric of Nylon for testing from the Clothing-Life Research Association was removed of water-soluble components in a mixed solution of methanol-water at a volume ratio of 1:1, and then the fabric was dried in the air, removed of oily components with benzene, and dried in the air. Thereafter, this was used as the testing fabric (white fabric, the same hereinafter).

0.2 g of the testing fabric was put into a vial, and 9.5 mL of a 50 mM Tris-HCl buffer solution (pH 9.0) containing the rice hull peroxidase (360 nM) and hydrogen peroxide (790 μM) were added thereto to a final bath ratio of 1:50, and stirring was initiated at 20°C. Then, 0.5 mL of a 1 mM aqueous Orange II solution was added to the vial, and the reaction was carried out at 20°C for 30 minutes. After the reaction, the testing fabric was taken and dried in the air. Then, in order to indicate the degree of coloration of Orange II to the testing fabric after the experiment for prevention of dye transfer, the color differences (ΔE) were measured, using a Minolta Colorimeter CR-300.

Here, the term color difference (ΔE) described herein is not relevant to the three inherent properties of hue, brightness and chromaticity as indicated in the sensational color indication by the Committee of International Commission on Illumination (CIE), but it indicates the value defined to indicate quantitatively the sensation of the color. That is, the parameters for the color difference (ΔE), i.e., L, a and b are measured using a colorimeter, and ΔE can be calculated by a Hunter's equation for color difference as represented below

(Equation 2).

$$\Delta E = [((L)2 + ((a)2 + ((b)2]1/2$$

(L = L0 - Lw, (a = a0 - aw, (b = b0 - bw

wherein L0, a0 and b0 indicate the L, a and b values of the testing fabric before treatment, respectively, and Lw, aw and bw indicate the L, a and b values of the testing fabric after the treatment, respectively.

Further, as a comparative example, the same investigation was carried out with a system not containing the rice hull peroxidase. Moreover, an investigation was carried out with the above-described system, the system now containing alkylbenzene sulfonate (LAS: an anionic surfactant) or sodium laurate (a soap) as the surfactants at concentrations of 7.35 mM or 10.1 mM, respectively. The results of each experimental section are summarized in the following Table 11.

As shown below, dye transfer of Orange II to the testing fabric was prevented by the rice hull peroxidase. Further, the effect of the prevention of dye transfer in the presence of LAS or sodium laurate was also confirmed.

Table 11

| Rice Hull Peroxidase | Surfactant | Color Difference ((E) |
|---|---|---|
| Added | Not Added | 1.19 ± 0.38 |
| Not Added | Not Added | 10.60 ± 0.58 |
| Added | LAS | 0.65 ± 0.08 |
| Added | Sodium Laurate | 0.61 ± 0.03 |

EXAMPLE 10

Effect of the prevention of dye transfer to testing fabric (2)

An investigation was made on the effect of pH on the prevention of dye transfer to testing fabric by means of the rice hull peroxidase in a system using Orange II as the model textile dye.

Nylon fabric (Nylon 6) from Kansai Clothing-Life Research Association was removed of water-soluble components in a mixed solution of methanol-water at a volume ratio of 1:1, and then the fabric was dried in the air, removed of oily components with benzene, and dried in the air. Thereafter, this was used as the testing fabric (white fabric, the same hereinafter).

0.2 g of the testing fabric was put into a vial, and any one of 9.5 mL of an aqueous hydrogen peroxide solution (790 (M), 9.5 mL of a 50 mM Tris-HCl buffer solution (pH 9.0) containing hydrogen peroxide (790 (M) and 9.5 mL of a Carmody buffer solution (pH 10.0) containing hydrogen peroxide (790 (M), each having the rice hull peroxidase (360 nM), was added thereto to a final bath ratio of 1:50, and stirring was initiated at 20(C. Then, 0.5 mL of a 1 mM aqueous Orange II solution was added to the vial, and the reaction was carried out at 20(C for 30 minutes.

After the reaction, the testing fabric was taken and dried in the air, and the color differences ((E) were measured as in Example 9 to obtain the results as presented in Table 12 below. Here, as a comparative example, the same investigation was carried out with a system not containing SDS or the rice hull peroxidase.

As a result, the dye transfer of Orange II to the testing fabric was prevented by the addition of the rice hull peroxidase. The effect of pH was not recognized.

Table 12

| Rice Hull Peroxidase | Buffer Solution | Color Difference ((E) |
|---|---|---|
| Not Added | None | 30.10 |
| Not Added | 50 mM Tris-HCl buffer solution (pH 9.0) | 21.52 |
| Added | ditto | 4.47 |
| Not Added | Carmody buffer solution (pH 10.0) | 21.01 |
| Added | ditto | 2.47 |

EXAMPLE 11

Effect of the prevention of dye transfer to testing fabric (3)

An investigation was made on the effect of the prevention of dye transfer to testing fabric by the rice hull peroxidase in the presence of SDS in a system using Orange II as the model textile dye.

0.2 g of the testing fabric prepared in Example 9 was put into a vial, and 9.5 mL of a 50 mM Tris-HCl buffer solution (pH 9.0) containing the rice hull peroxidase (360 nM), sodium dodecyl sulfate (SDS: 9.0 mM) and hydrogen peroxide (790 μM) was added thereto to a final bath ratio of 1:50, and stirring was initiated at 20°C or 60°C. Then, 0.5 mL of a 1 mM aqueous Orange II solution was added to the vial, and the reaction was carried out at 20°C or 60°C for 30 minutes.

After the reaction, the testing fabric was taken and dried in the air. The color differences ($\Delta E$) were measured as in Example 9 to obtain results as described in Table 13. Here, as comparative examples, the same examinations were carried out in a system not containing SDS or the rice hull peroxidase.

As a result, the dye transfer of Orange II to the testing fabric was prevented by the addition of the rice hull peroxidase. Also, in the presence of SDS, the synergistic effect of dye transfer prevention was confirmed.

Table 13

| Rice hull peroxidase | SDS | Color Difference ($\Delta E$) : 20°C | Color Difference ($\Delta E$) : 60°C |
|---|---|---|---|
| Added | Added | 0.35 ± 0.02 | 0.91 ± 0.45 |
| Added | Not Added | 1.19 ± 0.38 | 2.43 ± 0.15 |
| Not Added | Added | 2.30 ± 0.33 | 4.92 ± 0.32 |
| Not Added | Not Added | 10.60 ± 0.58 | Data Not Available |

EXAMPLE 12

Effect of the prevention of dye transfer to testing fabric (4)

An investigation was made on the effect of the prevention of dye transfer to testing fabric by the rice hull peroxidase in the presence of Brij35 in a system using Orange II as the model textile dye.

0.2 g of the testing fabric prepared in Example 9 was put into a vial, and 9.5 mL of a 50 mM Tris-HCl buffer solution (pH 9.0) containing the rice hull peroxidase (360 nM), Brij35 (95 μM) and hydrogen peroxide (790 μM) was added thereto to a final bath ratio of 1:50, and stirring was initiated at 20°C or 60°C. Then, 0.5 mL of a 1 mM aqueous Orange II solution was added to the vial, and the reaction was carried out at 20°C or 60°C for 30 minutes.

After the reaction, the testing fabric was taken and dried in the air. The color differences ($\Delta E$) were measured as in Example 9 to obtain results as described in Table 14. Here, as a comparative example, the same investigation was carried out in a system not containing the rice hull peroxidase.

Thus, the effect of dye transfer prevention of Orange II to the testing fabric by the rice hull peroxidase in the presence of Brij35 was confirmed.

Table 14

| Rice Hull Peroxidase | Color Difference (ΔE): 20°C | Color Difference (ΔE): 60°C |
|---|---|---|
| Added | 0.96 ± 0.09 | 3.71 ± 0.42 |
| Not Added | 7.18 ± 0.61 | 19.10 ± 0.67 |

EXAMPLE 13

Effect of the prevention of dye transfer to testing fabric (5)

An investigation was made on the effect of the prevention of dye transfer to testing fabric by the rice hull peroxidase in the presence of sodium percarbonate in a system using Orange II as the model textile dye.

0.2 g of the testing fabric prepared in Example 10 was put into a vial, and 9.5 mL of a 50 mM Tris-HCl buffer solution (pH 9.0) containing the rice hull peroxidase (360 nM), sodium percarbonate (105 mM) and hydrogen peroxide (790 μM) was added thereto to a final bath ratio of 1:50, and stirring was initiated at 20°C or 40°C. Then, 0.5 mL of a 1 mM aqueous Orange II solution was added to the vial, and the reaction was carried out at 20°C or 40°C for 30 minutes.

After the reaction, the testing fabric was taken and dried in the air. The color differences (ΔE) were measured as in Example 9 to obtain results as described in Table 15. As a comparative example, the same investigation was carried out in a system not containing the rice hull peroxidase.

Thus, the effect of dye transfer prevention of Orange II to the testing fabric by the rice hull peroxidase in the presence of sodium percarbonate was confirmed.

Table 15

| Rice Hull Peroxidase | Sodium Percarbonate | Hydrogen Peroxide | Temperature | Color Difference (ΔE) |
|---|---|---|---|---|
| Not Added | Not Added | Not Added | 20°C | 21.52 |
| Not Added | Added | Not Added | Ditto | 9.23 |
| Added | Not Added | Added | Ditto | 4.47 |
| Added | Added | Not Added | Ditto | 1.55 |
| Not Added | Not Added | Not Added | 40°C | 23.76 |
| Not Added | Added | Not Added | Ditto | 2.12 |
| Added | Not Added | Added | Ditto | 6.33 |
| Added | Added | Not Added | Ditto | 1.20 |

EXAMPLE 14

Effect of the prevention of dye transfer in a model detergent system (1)

An investigation was made on the effect of the prevention of dye transfer to testing fabric by the rice hull peroxidase in the presence of a model detergent component in a system using Orange II as the model textile dye.

0.2 g of the testing fabric prepared in Example 9 was put into a vial, and 9.5 mL of water having a mixture of the rice hull peroxidase (360 nM), hydrogen peroxide (790 μM) and a model detergent component (the composition and the final concentration were the same as those of Example 8) was added thereto to a final bath ratio of 1:50, and stirring was initiated at 20°C. Then, 0.5 mL of a 1 mM aqueous Orange II solution was added to the vial, and the reaction was carried out at 20°C for 30 minutes.

After the reaction, the testing fabric was taken and dried in the air. The color differences (ΔE) were measured as in Example 9 to obtain results as described in Table 16. As a comparative example, the same investigation was carried out in a system containing neither the rice hull peroxidase nor the model detergent component (a simple water system).

Thus, the effect of dye transfer prevention of Orange II to the testing fabric by the rice hull peroxidase in the presence of a model detergent component was confirmed.

Table 16

| Rice Hull Peroxidase | Model Detergent Component | Color Difference (ΔE) |
|---|---|---|
| Added | Added | 0.27 ± 0.04 |
| Not Added | Not Added | 13.70 ± 0.61 |

EXAMPLE 15

Effect of the prevention of dye transfer in a model detergent system (2)

An investigation was made on the effect of the prevention of dye transfer to testing fabric by the rice hull peroxidase in the presence of a model detergent component containing a bleaching activator, in a system using Orange II as the model textile dye.

0.2 g of the testing fabric prepared in Example 10 was put into a vial, and 9.5 mL of water having a mixture of the rice hull peroxidase (360 nM), sodium percarbonate (105 mM), a model detergent component (the composition and the final concentration were the same as those of Example 8) and a bleaching activator (a sodium salt of nonanoyloxybenzenesulfonic acid, or a sodium salt of dodecanoyloxybenzenesulfonic acid, or 4-decanoyloxybenzoic acid; the concentration of each being 52 mM) was added thereto to a final bath ratio of 1:50, and stirring was initiated at 20°C. Then, 0.5 mL of a 1 mM aqueous Orange II solution was added to the vial, and the reaction was carried out at 20°C for 30 minutes.

After the reaction, the testing fabric was taken and dried in the air. The color differences (ΔE) were measured as in Example 9 to obtain results as described in Table 17. Thus, the effect of dye transfer prevention of Orange II to the testing fabric by the rice hull peroxidase in the presence of a bleaching activator was confirmed.

Table 17

| Bleaching Activator | Color Difference (ΔE) |
|---|---|
| Not Added | 1.67 |
| Sodium Salt of Nonanoyloxybenzenesulfonic acid | 0.43 |
| Sodium Salt of Dodecanoyloxybenzenesulfonic acid | 0.51 |
| 4-Decanoyloxybenzoic acid | 0.58 |

EXAMPLE 16

Effect of the prevention of dye transfer to testing fabric (6)

An investigation was made on the effect of the prevention of dye transfer to testing fabric by the rice hull peroxidase in a system using a reactive dye Cibacron Blue F3G-A (C.I. Reactive Blue 2: available from Fluka Bioceika) as the model textile dye.

Cotton fabric for washing test designated by the Japanese Oil Chemists' Society (Calico 2023) was removed of water-soluble components in a mixed solution of methanol-water at a volume ratio of 1:1, and then the fabric was dried in the air, removed of oily components with benzene, and dried in the air. Thereafter, this was used as the testing fabric.

0.2 g of the Nylon testing fabric prepared in Example 10 or 0.2 g of the above-described cotton testing fabric was put into a vial, and 9.5 mL of a 50 mM Tris-HCl buffer solution (pH 9.0) containing the rice hull peroxidase (360 nM), p-iodophenol (52 μM), and sodium percarbonate (105 mM) or hydrogen peroxide (790 μM) was added thereto to a final bath ratio of 1:50, and stirring was initiated at 20°C or 40°C. Then, 0.5 mL of a 1 mM aqueous Cibacron Blue F3G-A solution was added to the vial, and the reaction was carried out at 20°C or 40°C for 30 minutes.

After the reaction, the testing fabric was taken and dried in the air, and the color differences (ΔE) were measured as in Example 9 to obtain the results as presented in Table 18 and Table 19 below.

Here, as comparative examples, the same investigation was carried out with a system containing no rice hull peroxidase, a system containing no sodium percarbonate or a system using water instead of the 50 mM Tris-HCl buffer solution.

Table 18

| Results in Nylon testing fabric | | | | |
|---|---|---|---|---|
| Rice Hull Peroxidase | Hydrogen Peroxide Source | Tris Buffer Solution | Temperature | Color Difference ($\Delta$E) |
| Not Added | Not Added | Not Used (Water) | 20°C | 10.31 |
| Not Added | Not Added | Used | Ditto | 10.41 |
| Not Added | Sodium Percarbonate | Used | Ditto | 2.84 |
| Added | Hydrogen Peroxide | Used | Ditto | 1.10 |
| Added | Sodium Percarbonate | Used | Ditto | 1.38 |
| Not Added | Not Added | Not Used (Water) | 40°C | 17.38 |
| Not Added | Not Added | Used | Ditto | 13.79 |
| Added | Hydrogen Peroxide | Used | Ditto | 4.01 |

Table 19

| Results in cotton testing fabric | | | | |
|---|---|---|---|---|
| Rice Hull Peroxidase | Hydrogen Peroxide Source | Tris Buffer Solution | Temperature | Color Difference ($\Delta$E) |
| Not Added | Not Added | Not Used (Water) | 20°C | 1.39 |
| Not Added | Not Added | Used | Ditto | 7.86 |
| Not Added | Sodium Percarbonate | Used | Ditto | 18.82 |
| Added | Hydrogen Peroxide | Used | Ditto | 1.51 |
| Added | Sodium Percarbonate | Used | Ditto | 5.54 |
| Not Added | Not Added | Not Used (Water) | 40°C | 1.38 |
| Not Added | Not Added | Used | Ditto | 9.07 |
| Added | Hydrogen Peroxide | Used | Ditto | 2.86 |

EXAMPLE 17

Effect of the prevention of dye transfer to testing fabric (7)

An investigation was made on the effect of pH affecting the effect of the prevention of dye transfer to testing fabric by the rice hull peroxidase in a system using a reactive dye, Cibacron Blue F3G-A as the model textile dye.

0.2 g of the Nylon testing fabric prepared in Example 10 or 0.2 g of the cotton testing fabric prepared in Example 16 was put into a vial, and 9.5 mL of a Carmody buffer solution (pH 10.0) containing the rice hull peroxidase (360 nM), p-iodophenol (50 $\mu$M) and hydrogen peroxide (790 $\mu$M) was added thereto to a final bath ratio of 1:50, and stirring was initiated at 20°C or 40°C. Then, 0.5 mL of a 1 mM aqueous Cibacron Blue F3G-A solution was added to the vial, and the reaction was carried out at 20°C or 40°C for 30 minutes.

After the reaction, the testing fabric was taken and dried in the air. The color differences ($\Delta$E) were measured as in Example 9 to obtain results as described in Table 20.

Here, as a comparative example, the same investigation was carried out in a system containing no rice hull peroxidase.

Table 20

| Rice Hull Peroxidase | Testing Fabric | Temperature | Color Difference ($\Delta$E) |
|---|---|---|---|
| Not Added | Nylon | 20°C | 4.33 |

Table 20   (continued)

| Rice Hull Peroxidase | Testing Fabric | Temperature | Color Difference (ΔE) |
|---|---|---|---|
| Not Added | Ditto | Ditto | 2.63 |
| Not Added | Ditto | 40°C | 8.92 |
| Added | Ditto | Ditto | 2.15 |
| Added | Cotton | 20°C | 17.16 |
| Not Added | Ditto | Ditto | 5.48 |
| Not Added | Ditto | 40°C | 15.41 |
| Added | Ditto | Ditto | 4.01 |

EXAMPLE 18

Color fading of dyed fabric (1)

An investigation was made on the discoloration of dyed fabric by using the rice hull peroxidase in the case of using Orange II as the model textile dye, with the washing temperature set at 20°C.

Nylon testing fabric from the Clothing-Life Research Association was placed in an aqueous solution containing 2% of Orange II (with respect to the textile weight), 10% of acetic acid (with respect to the textile weight) and 10% of anhydrous sodium sulfate (with respect to the textile weight) having a bath ratio of 1:50, and the temperature of the aqueous solution was elevated from room temperature to 90°C for 15 minutes. Then, the fabric was subjected to dyeing for 15 minutes, rinsed with water and dried in the air to obtain dyed fabric (original fabric).

10.0 mL of an aqueous solution containing hydrogen peroxide (750 µM as a final concentration) and the components listed in Table 21 below was added to a vial, and stirring was initiated at 20°C. Then, 0.2 g of the testing fabric (to a final bath ratio of 1:50) was put into the vial, and stirring was continued at 20°C. After 30 minutes, the dyed fabric was taken and was measured for the color differences (ΔE) against the original fabric as in Example 9. The results are summarized in Table 21 below.

Thus, the rice hull peroxidase did not show any color fading on dyed fabric at 20°C.

Table 21

| Rice Hull Peroxidase (Final Conc.) | Surfactant (Final Conc.) | Buffer Component (Final Conc.) | Color Difference (ΔE) |
|---|---|---|---|
| Not Added | Not Added | Not Added (Water only) | 0.615 |
| Not Added | Not Added | Tris-HCI (50 mM) | 0.583 |
| Added (340 nM) | Not Added | Tris-HCI (50 mM) | 0.710 |
| Added (340 nM) | SDS (8.55 mM) | Tris-HCI (50 mM) | 0.601 |
| Added (340 nM) | Brij35 (90 µM) | Tris-HCI (50 mM) | 0.767 |
| Added (340 nM) | Model Detergent Component (see Example 7) | Not Added (Water only) | 0.275 |

EXAMPLE 19

Color fading of dyed fabric (2)

An investigation was made on the color fading of dyed fabric by the rice hull peroxidase in the case of using Orange II as the model textile dye, with the washing temperature set at 60°C.

10.0 mL of an aqueous solution containing hydrogen peroxide (750 µM as a final concentration) and the components listed in Table 22 below was added to a vial, and stirring was initiated at 60°C. Then, 0.2 g of the testing fabric as prepared in Example 18 (to a final bath ratio of 1:50) was put into the vial, and stirring was continued at 60°C. After 30 minutes, the dyed fabric was taken and was measured for the color differences (ΔE) against the original fabric as in Example 9. The results are summarized in Table 22 below.

As a result, at 60°C, color fading of the dyed fabric occurred in the solutions containing SDS and the Tris-HCl buffer component, whereas addition of the rice hull peroxidase did not show any increase in the color fading on the dyed fabric.

Table 22

| Rice Hull Peroxidase (Final Conc.) | Surfactant (Final Conc.) | Buffer Component (Final Conc.) | Color Difference (ΔE) |
|---|---|---|---|
| Not Added | Not Added | Not Added (Water only) | 1.23 |
| Not Added | Not Added | Tris-HCl (50 mM) | 14.43 |
| Added (340 nM) | Not Added | Tris-HCL (50 mM) | 14.51 |
| Not Added | SDS (8.55 mM) | Tris-HCl (50 mM) | 18.00 |
| Added (340 nM) | SDS (8.55 mM) | Tris-HCl (50 mM) | 17.88 |

EXAMPLE 20

Prevention of dye transfer from dyed fabric to white fabric (1) An investigation was made on the prevention of dye transfer from dyed fabric to white fabric by the rice hull peroxidase in the case of using Orange II as the model textile dye, with the washing temperature set at 20°C.

20.0 mL of a 50 mM Tris-HCl buffer solution (pH 9.0) containing the rice hull peroxidase (360 nM), and sodium percarbonate (105 mM) or hydrogen peroxide (790 μM) was added to a vial, and stirring was initiated at 20°C. Then, 0.2 g of the dyed fabric as prepared in Example 18 (to a final bath ratio of 1:50) and 0.2 g of the testing fabric as prepared in Example 10 (white fabric) were put into the vial, and stirring was continued at 20°C. After 30 minutes, the dyed fabric and the testing fabric were taken, and their color differences (ΔE) against the original fabric were measured as in Example 9. Here, as comparative examples, the same investigation was carried out on a system containing no rice hull peroxidase, a system containing no sodium percarbonate and a system using water instead of 50 mM Tris-HCl buffer solution.
The results are summarized in Table 23 and Table 24 below. Thus, the effect of the prevention of dye transfer from dyed fabric to white fabric by the rice hull peroxidase was confirmed.

Table 23

| Color change or discoloration of dyed fabric (Color differences due to discoloration) | | | |
|---|---|---|---|
| Rice Hull Peroxidase | Hydrogen Peroxide Source | Tris Buffer Solution | Color Difference (ΔE) |
| Not Added | Not Added | Not Used (Water) | 0.90 |
| Not Added | Not Added | Used | 2.39 |
| Added | Hydrogen Peroxide | Used | 3.39 |
| Not Added | Sodium Percarbonate | Used | 2.09 |
| Added | Sodium Percarbonate | Used | 2.31 |

Table 24

| Color change of white fabric (Color differences due to coloration) | | | |
|---|---|---|---|
| Rice Hull Peroxidase | Hydrogen Peroxide Source | Tris Buffer Solution | Color Difference (ΔE) |
| Not Added | Not Added | Not Used (Water) | 34.63 |
| Not Added | Not Added | Used | 37.20 |
| Added | Hydrogen Peroxide | Used | 23.06 |
| Not Added | Sodium Percarbonate | Used | 30.59 |
| Added | Sodium Percarbonate | Used | 21.85 |

EXAMPLE 21

Prevention of dye transfer from dyed fabric to white fabric (2)

An investigation was made on the prevention of dye transfer from dyed fabric to white fabric by the rice hull peroxidase in the presence of a bleaching activator in the case of using Orange II as the model textile dye, with the washing temperature set at 20°C.

20.0 mL of a 50 mM Tris-HCl buffer solution (pH 9.0) containing the rice hull peroxidase (360 nM), sodium percarbonate (105 mM), and a bleaching activator (a sodium salt of nonaoyloxybenzenesulfonic acid, or a sodium salt of dodecanoyloxybenzenesulfonic acid, or 4-decanoyloxybenzoic acid; the concentration of each being 52 mM) was added to a vial, and stirring was initiated at 20°C. Then, 0.2 g of the dyed fabric as prepared in Example 18 (to a final bath ratio of 1:50) and 0.2 g of the testing fabric as prepared in Example 10 (white fabric) were put into the vial, and stirring was continued at 20°C. After 30 minutes, the dyed fabric and the testing fabric were taken, and their color differences (ΔE) against the original fabric were measured as in Example 9.

The results are summarized in Table 25 and Table 26 below. Thus, the effect of the prevention of dye transfer from dyed fabric to white fabric by the rice hull peroxidase in the presence of a bleaching activator was confirmed.

Table 25

| Color change or discoloration of dyed fabric (Color differences due to discoloration) | |
| --- | --- |
| Bleaching Activator | Color Difference (ΔE) |
| Not Added | 2.53 |
| Sodium Salt of Nonanoyloxybenzenesulfonic acid | 2.46 |
| Sodium Salt of Dodecanoyloxybenzenesulfonic acid | 2.35 |
| 4-Decanoyloxybenzoic acid | 2.62 |

Table 26

| Color change of white fabric (Color differences due to coloration) | |
| --- | --- |
| Bleaching Activator | Color Difference (ΔE) |
| Not Added | 22.53 |
| Sodium Salt of Nonanoyloxybenzenesulfonic acid | 8.56 |
| Sodium Salt of Dodecanoyloxybenzenesulfonic acid | 9.43 |
| 4-decanoyloxybenzoic acid | 10.35 |

EXAMPLE 22

Prevention of dye transfer from dyed fabric to white fabric (3)

An investigation was made on the prevention of dye transfer from dyed fabric to white fabric by the rice hull peroxidase in the case of using a reactive dye, Cibacron Blue F3G-A as the model textile dye, with the washing temperature set at 20°C.

Cotton testing fabric (Calico 2023) designated by the Japanese Emulsion Society was placed in an aqueous solution containing Cibacron Blue F3G-A (2% with respect to the textile weight) and anhydrous sodium sulfate (60 g/L) having a bath ratio of 1:30, and the temperature of the aqueous solution was elevated from room temperature to 40°C for 5 minutes. Then, the cotton fabric was subjected to dyeing for 25 minutes, taken, and lightly dewatered. In order for the concentration of sodium carbonate in the remaining dye solution to be 60 g/L, the cotton fabric was repeatedly immersed in the solution. The temperature was elevated from 75°C to 80°C for 20 minutes, and then the fabric was dyed for 30 minutes. The cotton fabric was taken again, rinsed, and dried in the air to obtain dyed fabric (original fabric).

30.0 mL of a 50 mM Tris-HCl buffer solution (pH 9.0) containing the rice hull peroxidase (360 nM), p-iodophenol (52 μM), and sodium percarbonate (105 mM) or hydrogen peroxide (790 μM) was added to a vial, and stirring was initiated at 20°C. Then, 0.2 g of thus prepared dyed fabric (to a final bath ratio of 1:50), 0.2 g of the Nylon testing fabric (white fabric) as prepared in Example 10 and 0.2 g of the cotton testing fabric (white fabric) as prepared in Example 16 were put into the vial, and stirring was continued at 20°C. After 30 minutes, the dyed fabric and the testing fabric were taken, and their color differences (ΔE) against the original fabric were measured as in Example 9. Here, as comparative examples, the same investigation was carried out with a system containing no rice hull peroxidase, a system containing no sodium percarbonate, a system using water or a Carmody buffer solution (pH 10.0) instead of the 50 mM Tris-HCl buffer solution.

The results are summarized in Table 27, Table 28 and Table 29 below. Thus, the effect of the prevention of dye transfer from dyed fabric to white fabric by the rice hull peroxidase was also confirmed with respect to a reactive dye.

Table 27

| Color change or discoloration of dyed fabric (Color differences due to discoloration) | | | |
|---|---|---|---|
| Rice Hull Peroxidase | Hydrogen Peroxide Source | Type of Buffer Solution | Color Difference (ΔE) |
| Not Added | Not Added | Not Used (Water) | 2.91 |
| Not Added | Not Added | Carmody Buffer Soln. | 2.95 |
| Added | Hydrogen Peroxide | Carmody Buffer Soln. | 2.78 |
| Not Added | Not Added | Tris Buffer Solution | 3.36 |
| Added | Hydrogen Peroxide | Tris Buffer Solution | 3.67 |
| Not Added | Sodium Percarbonate | Tris Buffer Solution | 2.91 |
| Added | Sodium Percarbonate | Tris Buffer Solution | 2.93 |

Table 28

| Color change of Nylon white fabric (Color differences due to coloration) | | | |
|---|---|---|---|
| Rice Hull Peroxidase | Hydrogen Peroxide Source | Type of Buffer Solution | Color Difference (ΔE) |
| Not Added | Not Added | Not Used (Water) | 16.19 |
| Not Added | Not Added | Carmody Buffer Soln. | 3.66 |
| Added | Hydrogen Peroxide | Carmody Buffer Soln. | 1.73 |
| Not Added | Not Added | Tris Buffer Solution | 7.04 |
| Added | Hydrogen Peroxide | Tris Buffer Solution | 3.19 |
| Not Added | Sodium Percarbonate | Tris Buffer Solution | 4.22 |
| Added | Sodium Percarbonate | Tris Buffer Solution | 3.18 |

Table 29

| Color change of cotton white fabric (Color differences due to coloration) | | | |
|---|---|---|---|
| Rice Hull Peroxidase | Hydrogen Peroxide Source | Type of Buffer Solution | Color Difference (ΔE) |
| Not Added | Not Added | Not Used (Water) | 1.47 |
| Not Added | Not Added | Carmody Buffer Soln. | 7.08 |
| Added | Hydrogen Peroxide | Carmody Buffer Soln. | 1.75 |
| Not Added | Not Added | Tris Buffer Solution | 3.22 |
| Added | Hydrogen Peroxide | Tris Buffer Solution | 1.49 |
| Not Added | Sodium Percarbonate | Tris Buffer Solution | 9.92 |
| Added | Sodium Percarbonate | Tris Buffer Solution | 5.50 |

Industrial Applicability

The rice peroxidase (more specifically, rice hull peroxidase) according to the invention is excellent in reactivity and stability at high temperatures. Therefore, the invention provides a method of preventing the transfer of a textile dye from one dyed fabric to another fabric in the case of washing and/or rinsing a plurality of fabrics including dyed fabric together in a washing liquor, by using the rice peroxidase.

Further, the invention provides a method of bleaching a textile dye in a solution or a dispersion solution by using a rice peroxidase.

Furthermore, the invention provides a composition for decomposing (discoloring □ bleaching) of a coloring substance (a dye, etc.) in a solution by using a rice peroxidase.

**Claims**

1. A method of preventing the transfer of a textile dye from one dyed fabric to another fabric in the case of washing and/or rinsing a plurality of fabrics including dyed fabric together in a washing liquor, wherein a rice peroxidase acts on the washing liquor in the presence of hydrogen peroxide.

2. A method of bleaching a textile dye in a solution or a dispersion solution, wherein a rice peroxidase acts on the solution or dispersion solution in the presence of hydrogen peroxide.

3. The method according to claim 1 or 2, wherein the rice peroxidase is a heat-resistant rice peroxidase.

4. The method according to any one of claims 1 to 3, wherein the ricef peroxidase is a rice hull peroxidase.

5. The method according to any one of claims 1 to 4, wherein the hydrogen peroxide is supplied by at least one means of a hydrogen peroxide precursor, a combined system of a hydrogen peroxide precursor and of a bleaching activator which can generate hydrogen peroxide by acting on the hydrogen peroxide precursor, and an enzyme system which can generate hydrogen peroxide.

6. The method according to claim 5, wherein the hydrogen peroxide precursor is perborate or percarbonate.

7. The method according to any one of claims 1 to 6, which is carried out in the presence of an activator.

8. The method according to any one of claims 1 to 7, which is carried out in the presence of a surfactant.

9. The method according to any one of claims 1 to 8, wherein the textile dye is a synthetic dye, a natural dye, a natural-equivalent dye or a reactive dye.

10. A composition comprising a rice peroxidase.

11. The composition according to claim 10, which is a detergent composition or a bleaching composition.

12. The composition according to claim 10 or 11, which comprises at least one of hydrogen peroxide, one or more kinds of hydrogen peroxide precursors, and one or more kinds of enzyme systems which can generate hydrogen peroxide.

13. A composition which comprises a rice peroxidase, a hydrogen peroxide precursor, and one or more kinds of bleaching activators which can generate hydrogen peroxide by acting on the hydrogen peroxide precursor.

14. The composition according to any one of claims 10 to 13, which comprises one or more kinds of the surfactants and one or more kinds of the activators together.

FIGURE 1

Comparison of Various Peroxidases

FIGURE 2

Effect of Activator on RHP

**EP 1 550 711 A1**

## INTERNATIONAL SEARCH REPORT

| | International application No. |
|---|---|
| | PCT/JP03/12143 |

**A. CLASSIFICATION OF SUBJECT MATTER**
Int.Cl$^7$ C11D3/386, 3/395, C12N9/08

According to International Patent Classification (IPC) or to both national classification and IPC

**B. FIELDS SEARCHED**

Minimum documentation searched (classification system followed by classification symbols)
Int.Cl$^7$ C11D1/00-19/00, C12N9/08

Documentation searched other than minimum documentation to the extent that such documents are included in the fields searched

Electronic data base consulted during the international search (name of data base and, where practicable, search terms used)
CAPLUS(STN), REGISTRY(STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 7-91169 B2 (ASAHI CHEMICAL INDUSTRY CO., LTD.),<br>04 October, 1995 (04.10.95),<br>Claim 1; page 2, right column, line 49 to page 3, left column, line 10; examples 1 to 8<br>(a reference cited in the specification of the present application)<br>(Family: none) | 10<br>1-9,11-14 |

☒ Further documents are listed in the continuation of Box C.    ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 26 December, 2003 (26.12.03) | 27 January, 2004 (27.01.04) |

| Name and mailing address of the ISA/ | Authorized officer |
|---|---|
| Japanese Patent Office | |
| Facsimile No. | Telephone No. |

Form PCT/ISA/210 (second sheet) (July 1998)

**INTERNATIONAL SEARCH REPORT**

International application No.

PCT/JP03/12143

| C (Continuation). DOCUMENTS CONSIDERED TO BE RELEVANT | | |
|---|---|---|
| Category* | Citation of document, with indication, where appropriate, of the relevant passages | Relevant to claim No. |
| Y | JP 2801398 B2 (NOVO-NORDISK A/S),<br>10 July, 1998 (10.07.98),<br>Claim 1; page 4, left column, lines 13 to 34;<br>examples 1, 3, 9, 11 to 12 (a reference cited<br>in the specification of the present application)<br>& EP 495836 A1        & US 5273896 A1<br>& US 5605832 A1        & US 5648262 A1<br>& US 5700770 A1        & US 5712153 A1<br>& US 5855621 A1        & CN 1051600 A1<br>& BR 9007739 A1        & FI 9201315 A | 1-14 |
| Y | JP 10-500728 A (NOVO-NORDISK A/S),<br>20 January, 1998 (20.01.98),<br>Abstract; Claims 1, 6, 9; page 7, lines 1 to 9<br>& WO 95/33039 A1        & EP 763094 A1<br>& CN 1149314 A1        & BR 9507773 A<br>& FI 9604763 A | 1-14 |
| Y | JP 10-501274 A (PROCTER AND GAMBLE CO.),<br>03 February, 1998 (03.02.98),<br>Abstract; Claim 1; page 5, 16th line from the<br>bottom to 14th line from the bottom<br>& WO 95/33040 A1        & US 5451337 A1<br>& EP 763093 A1        & CN 1154139 A1<br>& BR 9507808 A | 1-14 |

Form PCT/ISA/210 (continuation of second sheet) (July 1998)

## INTERNATIONAL SEARCH REPORT

International application No.

PCT/JP03/12143

| Box I    Observations where certain claims were found unsearchable (Continuation of item 2 of first sheet) |

This international search report has not been established in respect of certain claims under Article 17(2)(a) for the following reasons:

1. ☐ Claims Nos.:

because they relate to subject matter not required to be searched by this Authority, namely:

2. ☐ Claims Nos.:

because they relate to parts of the international application that do not comply with the prescribed requirements to such an extent that no meaningful international search can be carried out, specifically:

3. ☐ Claims Nos.:

because they are dependent claims and are not drafted in accordance with the second and third sentences of Rule 6.4(a).

| Box II    Observations where unity of invention is lacking (Continuation of item 3 of first sheet) |

This International Searching Authority found multiple inventions in this international application, as follows:

The matter common to claims 1 to 14 resides exclusively in "rice-origin peroxidase".

However, rice-origin peroxidase is disclosed in a document "JP 7-91169 B" and a composition (plant growth promoter) containing rice-origin peroxidase is also described therein.

Namely, the rice-origin peroxidase which is the common matter as described above falls within the category of prior art and, therefore, cannot be considered as "a special technical feature" in the meaning within the second sentence of PCT Rule 13.2. It is therefore obvious that claims 1 to 14 do not comply with the requirement of unity of invention. (Continued to extra sheet.)

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐    The additional search fees were accompanied by the applicant's protest.

☐    No protest accompanied the payment of additional search fees.

Form PCT/ISA/210 (continuation of first sheet (1)) (July 1998)

**INTERNATIONAL SEARCH REPORT**

International application No.

PCT/JP03/12143

Continuation of Box No. II of continuation of first sheet(1)

The inventions according to claim 1 and parts of claims 3 to 9 relate to the prevention of fiber dye migration with the use of the rice-origin peroxidase. The inventions according to claim 2 and parts of claims 3 to 9 relate to the bleaching of a fiber dye with the use of the rice-origin peroxidase. The inventions according to claims 10 to 14 relate to compositions containing the rice-origin peroxidase which are not restricted in use to the prevention of fiber dye migration or bleaching thereof.

Form PCT/ISA/210 (extra sheet) (July 1998)